# EUROPÄISCHE PATENTSCHRIFT

(11) **EP 1 626 636 B1**
(45) Veröffentlichungstag und Bekanntmachung des Hinweises auf die Patenterteilung: **27.07.2011**
(21) Anmeldenummer: 04726148.2
(22) Anmeldetag: 07.04.2004
(51) Int. Cl.: A44B 18/00

(54) **VERFAHREN ZUM HERSTELLEN EINES HAFTVERSCHLUSSTEILES**
METHOD FOR PRODUCING A CONTACT-FASTENER PART
PROCEDE DE REALISATION D'UNE PIECE DE FERMETURE ADHESIVE

(30) Priorität: 27.05.2003 DE 10325372
(43) Veröffentlichungstag der Anmeldung: 22.02.2006
(73) Patentinhaber: Gottlieb Binder GmbH & Co. KG, 71088 Holzgerlingen (DE)
(72) Erfinder: TUMA, Jan, 71083 Herrenberg-Mönchberg (DE); GERBER, Ingo, 71093 Weil im Schönbuch (DE)
(74) Vertreter: Bartels, Martin Erich Arthur
(86) Internationale Anmeldenummer: PCT/EP2004/003735
(87) Internationale Veröffentlichungsnummer: WO 2004/105536

(56) Entgegenhaltungen:
- WO-A-01/49776
- DE-C- 10 065 819

## Beschreibung

Die Erfindung betrifft ein Verfahren zum Herstellen eines Haftverschlußteiles, bei dem ein Träger mit einer Vielzahl von vorzugsweise einstückig mit ihm verbundenen Stengeln erstellt wird gemäß der Merkmalsausgestaltung des Oberbegriffes des Patentanspruches 1.

Ein Verfahren zum Herstellen eines Haftverschlußteils ist im Stand der Technik beispielsweise durch die PCT/WO 94/23610 aufgezeigt. Als Anwendungsmöglichkeit eines solcher Art hergestellten Haftverschlußteils wird insbesondere die Bildung eines Haftverschlusses für Babywindeln oder für Krankenhauskleidung offenbart. Um Haftverschlußteile, die in Haftverschlüssen derartiger Kleidungsstücke verwendet werden können, herzustellen, benötigt man eine relativ hohe Anzahl von Verhakungsmitteln pro Quadratzentimeter, was zu entsprechend hohen Produktionskosten führt, da die zur Bildung der Verhakungsmittel verwendete Formwalze im Stand der Technik die entsprechende Anzahl von offenen Hohlräumen aufweisen muss, die erst aufwendig einzuprägen sind. Die derart hergestellten Haftverschlußteile weisen auf einem folienartigen Trägermaterial einstückig angeordnete und in vertikaler Richtung vorstehende Stengel auf, die an ihrem freien Ende mit einer kopfseitigen Verbreiterung versehen sind, die zum Herstellen des lösbaren Haftverschlusses mit einem korrespondierenden Flausch- oder Schlaufenmaterial zusammenwirken, welches sich mechanisch mit der Unterseite der kopfartigen Verbreiterung des Stengelmaterials verhakt. Dahingehende Verschlußsysteme werden im deutschsprachigen Raum auch häufig mit der Markenbezeichnung Kletten^{®}-Haftverschlüsse beworben.

Nachteil dieser bekannten Kletten-Haftverschlußsysteme ist es, dass diese für eine wirksame Verhaftung, also zur Bildung des eigentlichen Haftverschlusses immer mit korrespondierenden Verschlußteilen (Flausch- oder Schlingenware) zusammenwirken müssen, wobei moderne Verschlußsysteme heute auch die Möglichkeit aufzeigen gleich ausgebildete Kopfverschlußteile miteinander unter Bildung des Haftverschlusses zu verbinden, indem die kopfseitigen Enden des einen Verschlußteiles in die Abstände zwischen die kopfseitigen Enden und Stengel des anderen Verschlußteiles eingreifen und die sich radial gegenüber den Stengeln verbreiterten Kopfteile verhaken sich dann mit ihren einander zugewandten Randflächen gegenseitig, wobei auch die dahingehenden Verschlußsysteme lösbar ausgestaltet sein können.

Des weiteren sind im Stand der Technik Verfahren zur Herstellung von fibrilierten Folien aus Polypropylen oder Polyethylen bekannt (DE 198 37 499 A1, US 6,432,347 B1), bei denen unter Verwendung der vorhandenen Anlagentechnik (Folienextrusionsanlagen für Flachfolie, Blasfolie oder zum Einsatz von Chill-Roll-Verfahren) durch spezielle Maßnahmen in der Technologie und an den Anlagen Folien ohne Aufteilung in Bändchen komplex gereckt, fibriliert und aufgewickelt werden und so eine netzartige Folie mit unterschiedlicher Breite und großer Länge herstellbar ist. Aufgrund des monoaxialen Reckens bei dieser bekannten Lösung kommt es zu einer verbesserten molekularen Faserorientierung und die derart herstellbaren Gewebe finden ihre Anwendung als Geotextilien oder im Bauwesen als Armierung. Ferner sind in der deutschen Auslegeschrift 1 175 385 sowie in dem US-Patent 6,432,347 eine Fibrilierung von Folienmaterial durch Einsatz einer Wirbeldüse bzw. mit einem gepulsten Wasserstrahl mit hohem Druck beschrieben, um dergestalt zu einem verbesserten Filtermaterial zu gelangen oder zu einem folienartigen Werkstoff mit verbesserten thermischen und akustischen Isolationseigenschaften.

Durch die WO 01/49776 A3 (CORRECTED VERSION) ist ein gattungsgemäßes Verfahren bekannt zum Herstellen eines Haftverschlußteils, bei dem ein Träger mit einer Vielzahl von mit ihm verbundenen Stengeln erstellt wird, wobei bei dem Haftverschlußteil zumindest ein Teil der freien Enden der Stengel mit einer Vielzahl von Einzelfasern versehen wird. Das mit dem bekannten Verfahren hergestellte Erzeugnis ist in Fig.7 der WO-Veröffentlichung aufgezeigt. Bei der bekannten Verfahrenslösung werden die einzelnen Stengel mittels eines Ätzverfahrens aus einem Grund-Substrat erhalten und bilden dann eine Art Nano-Röhrchenstruktur aus. Da durch das Ätzverfahren das Stengelmaterial auf seiner Oberseite angegriffen wird, können zur Erzielung einer homogenen Oberfläche diese mit einer Oxid- und/oder Nitritschicht versehen werden.

Ferner sind innerhalb der WO-Veröffentlichung noch Stengelteile zur Realisierung einer sog. Gecko-Fußstruktur oder Teilen derselben aufgezeigt, bei denen die Stengel selbst ausgehend von einem Trägersubstrat oder Trägerteil aus einer Vielzahl an Einzelfilamenten bestehen oder bei denen die Stengel als zylindrischer Körper massiv ausgebildet an ihrer Endseite jeweils eine Verbreiterung aufweisen, sei es in Form einer spatelartigen Auflagefläche, sei es in Form einer Kugelkopfstruktur. Für den Erhalt der dahingehend alternativen Stengelgeometrien werden neben dem bereits beschriebenen Ätzverfahren lithographische Auftragverfahren oder mechanische Formgebungsverfahren eingesetzt, bei denen vergleichbar einem Nano-Druckverfahren eine entsprechende Druckwalze mit Formvorsprüngen in das Grundsubstrat als Träger eingreift, um dergestalt zumindest ein Vorstadium für die gewünschten Stengelstrukturen zu erhalten. Neben diesen industriellen Formgebungsverfahren wird auch der Vorschlag unterbreitet, unmittelbar biologisches Grundmaterial, insbesondere in Form von Gecko-Fußteilen, für eine Produktapplikation einzusetzen. Mit den bekannten Verfahren ist eine Herstellung von Haftverschlußteilen im großindustriellen Maßstab nicht ohne weiteres möglich oder nur mit entsprechend hohem Kostenaufwand.

Ausgehend von diesem Stand der Technik, stellt sich die Erfindung die Aufgabe, die bekannten Verfahren dahingehend weiter zu verbessern, dass ein Haftverschlußteil erhalten ist, das zum einen funktionssicher im Gebrauch sowie kostengünstig in der Herstellung ist, und das in einer größeren Breite die Auswahl von zuordenbaren Komponenten ermöglicht, mit der das erfindungsgemäße Haftverschlußteil eine sichere Haftverbindung eingehen kann. Eine dahingehende Aufgabe löst ein Verfahren mit den Merkmalen des Patentanspruches 1 in seiner Gesamtheit.

Dadurch, dass gemäß dem kennzeichnenden Teil des Patentanspruches 1 der jeweilige Stengel mittels eines Formgebungsverfahrens unter Einsatz einer Siebwalze oder formwerkzeugfrei über ein Tröpfchen-Auftragverfahren erhalten wird, und dass anschließend der jeweilige Stengel an seinem freien Ende in eine Vielzahl von Einzelfasern aufgeteilt wird, ist eine Möglichkeit eröffnet, ein Haftverschlußteil sehr kostengünstig herzustellen. Mit dem erfindungsgemäßen Verfahren läßt sich der Durchmesser der jeweilige Faser sehr dünn wählen, so dass am freien Ende einer jeden Einzelfaser nur eine sehr kleine vorzugsweise eben ausgebildete Kontaktfläche zur Verfügung steht. Bei bevorzugten Ausgestaltungen kann der Dickenbereich dieser Strukturen im Nanometer-Bereich, beispielsweise bei 200 bis 500 nm liegen. Diese Größenordnungen genügen damit eine Wechselwirkung mit dem korrespondierenden Teil, an dem das Haftverschlußteil festgelegt werden soll über die sogenannten Van-der-Waals-Kräfte erfolgen kann. Diese sogenannten Van-der-Waals-Kräfte entstehen, weil sich die in einem Atom um den positiven Kern schwirrenden, negativ geladenen Elektronen kurzzeitig an einer Seite konzentrieren. Dadurch ist das Atom auf dieser Seite vorübergehend negativ, auf der anderen Seite hingegen positiv geladen. Das beeinflußt auch benachbarte Atome - in diesem Fall die Atome an den Enden der Einzelfasern eines jeden Stieles. Die Folge, eine Einzelfaser wird - je nachdem, welche Ladung sie abbekommt, entweder von den positiven oder den negativen Atomen der jeweiligen gegenüberliegenden Fläche angezogen. Je größer die Kontaktfläche in der Summe, um so stärker die auftretenden Kräfte. Obwohl die Van-der-Waals-Kräfte mit zu den schwächsten Kräften in der Natur zählen, genügt der Effekt, um zu relativ hohen Verschlußkräften zu gelangen. So kann beispielsweise eine sinnfällig ausgebildete Haftverschlußteilfläche in einer Größenordnung von mehreren Quadratzentimetern aufgrund der Van-der-Waals-Kräfte an den freien Enden der Einzelfasern eines jeden Stengels mehrere zig Kilo anheben oder stationär festlegen.

Der dahingehende Verschluß läßt sich dem Grunde nach mit jeder Oberfläche (Untergrund) lösbar verbinden, um dergestalt beispielsweise ein mit dem Haftverschlußteil verbundenes Karosserieteil mit einem Fahrzeugrahmen zu verbinden oder dergestalt ein Bild über das Haftverschlußteil an die Wand ohne weiteres Verbindungsmittel aufzuhängen, in dem das Haftverschlußteil mit seinen Einzelfasern direkt mit der Oberfläche der Wand über die Van-der-Waals-Kräfte wechselwirkt.

Sofern das nach dem erfindungsgemäßen Verfahren hergestellte Haftverschlußteil Eingang in die Bekleidungsindustrie findet, sind hier keine weiteren Modifikationen mehr notwendig, insbesondere ist an Teilen der Bekleidung kein Schlaufen- oder Flauschmaterial anzuordnen, um dergestalt eine Verhakung mit dem Haftverschlußteil sicherzustellen. Vielmehr kann hier unmittelbar das Haftverschlußteil mit den freien Enden der Einzelfasern mit dem Material des Bekleidungsstoffes zum Herstellen einer Verbindung wechselwirken. Es hat sich gezeigt, dass zum Lösen des Haftverschlußteiles von einer Oberfläche, dieses vorzugsweise von der Oberfläche am besten in einem Winkel von ca. 30° abgeschält wird, um dergestalt die Verhaftung über die Van-der-Waals-Kräfte zu lösen und den Verschluß von der Oberfläche beliebiger Natur wieder entfernen zu können. Die dahingehenden Anbringungs- und Lösevorgänge können in Abhängigkeit der Ausgestaltung des Verschlußsystemes mehrfach, vorzugsweise mehrere 1000 Mal erfolgen. Zum Herstellen des Verschlusses genügt es, das Haftverschlußteil mit den freien Enden der Einzelfasern seiner Stengel flächig an die Oberfläche aufzusetzen. Vorzugsweise ist dabei vorgesehen, dass die Länge der Einzelfilamente oder Fasern derart gewählt ist, dass diese für jeden Stengel an ihrem freien Ende in einer gemeinsamen Ebene münden, da die Van-der-Waals-Kräfte nur über eine ausgesprochen kurze Distanz wirken, so dass vorzugsweise vorzusehen ist, dass der Abstand der freien Kontaktenden der Einzelfilamente zu der vorgesehenen Oberfläche im wesentlichen gleich ausfällt. Um zu vermeiden, dass die Einzelfasern von der zu kontaktierenden Oberfläche wegknicken können, weisen diese vorzugsweise eine Länge auf, die weniger als 10 bis 30 % der gesamten Stengellänge beträgt. Hierdurch ist sichergestellt, dass der Stengel einen Gegenhalt für die Einzelfasern bildet und diese derart in ihrem Steifigkeitsverhalten unterstützt.

Sehr gute Verhaftungsresultate haben sich aus Versuchen ergeben, sofern etwa 4000 Stengel pro Quadratzentimeter Trägerfläche vorhanden sind, und etwa 500 Einzelfäden sich mit einer gemeinsamen Abschlußebene an jedem freien Ende eines derartigen Stengels befinden.

Bei einer bevorzugten Ausführungsform des erfindungsgemäßen Verfahrens werden zum Aufteilen der Einzelfasern aus den freien Enden der Stiele diese chemisch, mechanisch oder elektrisch (Wärme-) behandelt.

Weitere vorteilhafte Ausführungsformen des erfindungsgemäßen Verfahrens sind Gegenstand der sonstigen Unteransprüche.

Im Folgenden wird das erfindungsgemäße Verfahren anhand eines mit ihm erhaltenen Haftverschlußteiles nach der Zeichnung näher erläutert. Dabei zeigen in prinzipieller und nicht maßstäblicher Darstellung die
- Fig. 1: eine perspektivische Draufsicht auf einen Ausschnitt eines mit dem erfindungsgemäßen Verfahren hergestellten Teils, bestehend aus einem Trägermaterial und einer Vielzahl von auf ihm angeordneten, einstückig mit ihm verbundenen Stengeln;
- Fig. 2: eine Seitenansicht auf einen Ausschnitt aus dem Trägermaterial mit nur einem Stengel, mit an seinem freien Ende angeordneten Einzelfasern.

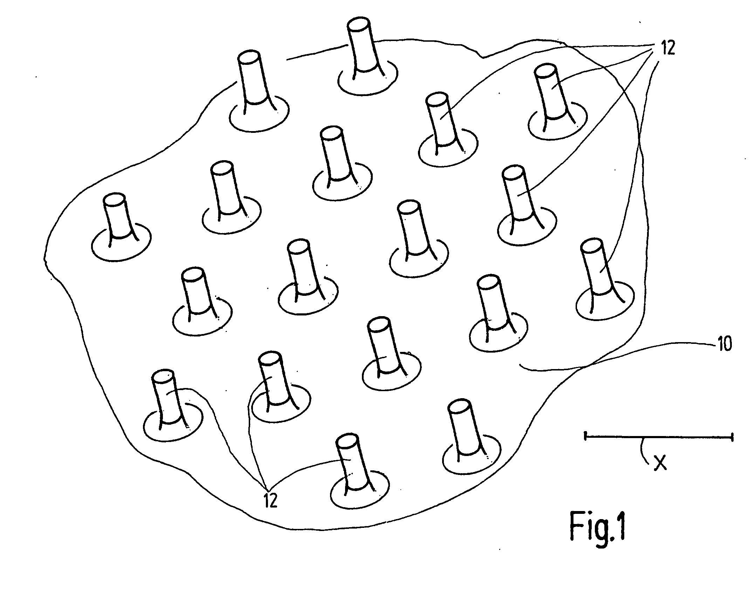

Das in der Fig. 1 gezeigte Ausgangsprodukt für ein Haftverschlußteil im Sinne dieser Erfindung läßt sich beispielsweise nach einem Verfahren wie in der DE 196 46 318 A1 beschrieben, erhalten. Das bekannte Verfahren dient der Herstellung eines Haftverschlußteiles mit einer Vielzahl von einstückig mit einem Trägermaterial 10 ausgebildeten Verhakungsmitteln in Form von Verdickungen aufweisenden Stengeln, bei dem ein vorzugsweise thermoplastischer Kunststoff in plastischem oder flüssigen Zustand, einem Spalt zwischen einer Druckwalze und einer Formwalze zugeführt wird, wobei die Formwalze mit einem Sieb versehen ist mit nach außen und innen offenen Hohlräumen, und beide Walzen werden für den Herstellvorgang im entgegengesetztem Drehsinn angetrieben, so dass das Trägermaterial im Spalt zwischen den Walzen gebildet wird. Da für das erfindungsgemäße Haftverschlußteil es genügt als Ausgangsprodukt Stengel 12 zu erhalten, wird das bekannte Herstellverfahren dahingehend modifiziert, dass sich in den Formhohlräumen des Siebes nur die Stengel 12 ausbilden und diese an ihrer freien Seite keine kopfförmigen Verdickungen in der Art von Verhakungsmitteln ausbilden. Dies läßt sich durch geeignete Wahl des Formhohlraumes erreichen oder durch Steuerung der Prozeßparameter. Wird beispielsweise das Fließverhalten des Kunststoffmateriales im Siebhohlraum gesteuert, läßt sich dergestalt das Fließverhalten des Kunststoffmateriales reduzieren, und aufgrund seiner Oberflächenspannung kommen dann Stengel 12 als Herstellprodukt zutage, deren freie Enden jeweils einen konvexen oder ebenen Abschluß aufweisen. Hierzu können die Sieb-Hohlräume auf einem niedrigen Temperaturniveau gehalten werden, beispielsweise gekühlt werden oder das Kunststoffmaterial wird mit geringerer Einspritztemperatur in die Formhohlräume eingebracht.

Eine andere Möglichkeit das in Fig. 1 dargestellte Stengel-Verschlußteil zu erhalten, ist in der DE 100 65 819 C1 beschrieben. Bei diesem bekannten Verfahren zum Herstellen von Haftverschlußteilen wird ein Trägermaterial in zumindest einem Teilbereich seiner Oberfläche mit aus deren Ebene hervorstehenden Haftverschlußelementen versehen, in dem ein die Haftverschlußelemente bildendes Kunststoffmaterial auf das Trägerelement aufgebracht wird, wobei die Haftverschlußelemente zumindest in einem Teilbereich formwerkzeugfrei ausgebildet sind, in dem das Kunststoffmaterial mittels mindestens einer Auftragevorrichtung in aufeinanderfolgend abgegebenen Tröpfchen abgelagert wird. Obwohl die Auftragevorrichtung über ihre Düse das Kunststoffmaterial mit einem Tröpfchenvolumen von nur wenigen Pikolitern ausbringt, läßt sich dergestalt ein derart schneller Prozeßablauf realisieren, dass in kürzester Zeit das Stengelmaterial nach der Fig. 1 erhalten ist. Auch hier wird bei Anwendung des Verfahrens darauf geachtet, dass über die Tröpfchen nur zylindrische Stengel 12 erhalten sind, mit im wesentlichen plan verlaufenden Kopfseiten. Auch besteht die Möglichkeit der Herstellung des in Fig. 1 gezeigten Stengelbandes dadurch, dass man beispielsweise in einer Formwalze spitz oder konisch zulaufende Stengelteile bei einer Temperatur im Bereich ihrer Formerweichung kopfseitig über nicht näher dargestellte Kalanderwalzen nachkalandriert, um dergestalt eben verlaufende Kopfenden für die einzelnen Stengel des Haftverschlußteiles zu erreichen.

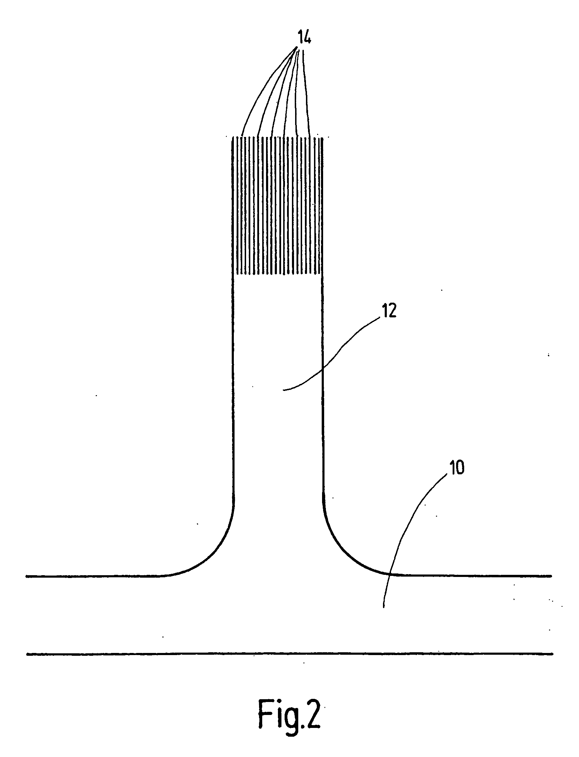

Mit dem genannten Verfahren lassen sich ohne weiteres 1000 bis 10.000 Stengel 12 pro Quadratzentimeter erreichen, wobei sich eine Stengelanzahl von etwa 4000 pro Quadratzentimeter Trägerfläche 10 als ausreichend erwiesen hat. Um eine Vorstellung von den Größenverhältnissen zu erhalten, ist in der Fig. 1 in Blickrichtung unten rechts eine Länge X aufgetragen, die der Größe 200 µm entspricht. Die Länge des in der Fig. 2 vergrößert wiedergegebenen Stengels gemessen vom freien Ende des Stengels bis zur Trägerfläche beträgt ca. 140 µm bei einem Durchmesser von ca. 33 µm, was einem Aspektverhältnis von etwa 5 : 1 gleichkommt.

Das dahingehende Stengelende wird in einem weiteren Herstellverfahrensschritt in eine Vielzahl von Einzelfasern 14 aufgeteilt, und vorzugsweise befinden sich etwa 500 Fäden mit einem Durchmesser von 0,2 µm an jedem Stengel 12. Ferner ist dafür Sorge getragen, dass die Länge der Einzelfasern 14 ca. 20 % der Gesamtlänge des Stengels 12 einschließlich den Einzelfasern 14 ausmachen. Dergestalt wird jede Einzelfaser 14 sicher von der sonstigen Stengellänge gehalten, und der sonstige Stengelquerschnitt bildet insoweit ein Widerlager für die Einzelfasern 14, um diese an einer vorgebbaren Oberfläche über die Van-der-Waals-Kräfte zu verhaften. Hierfür ist auch wichtig, dass die freien Enden der Einzelfasern 14 in einer Ebene zueinander liegen, die gemäß der Darstellung nach der Fig. 2 parallel zur Trägerfläche 10 des Trägermateriales für das Haftverschlußteil verläuft.

Der Träger 10, die einzelnen Stengel 12 und die Einzelfasern 14 eines jeden Stengels bestehen aus einem Kunststoffmaterial, das insbesondere ausgewählt ist aus der Gruppe der Acrylate wie Polymethacrylate, Polyethylen, Polypropylene, Polyoxymethylene, Polyvinyliden-fluorid, Polymethylpenten, Polyethylen-chlorotrifluoroethylen, Polyvinylfluorid, Polyethylenoxid, Polyethylenterephthalate, Polybutylenterephthalate, Nylon 6, Nylon 66 und Polybuten. Als besonders gut erweisen sich Kunststoffe mit langen Molekülketten und einem guten Orientierungsverhalten.

Um die Einzelfaser 14 nunmehr aus dem Stengel 12 zu erhalten, ist es notwendig in einem Herstellverfahrensschritt auf die dahingehenden Enden der Stiele 12 einzuwirken. Als mechanische Aufteifmöglichkeit besteht die Möglichkeit mit Schneidemessern, beispielsweise keramischen Mikromessern, die Stielenden über eine vorgebbare Länge aufzuschneiden, um dergestalt die Einzelfasern 14 zu erhalten. Andere Möglichkeiten sehen ein Aufbürsten oder Aufpeitschen der Stengelenden vor, um zu den Einzelfilamenten oder Fasern zu gelangen. Als mechanische Trennmittel kommen auch gepulste Wasserstrahlen in Frage, und des weiteren besteht die Möglichkeit über spezielle Laseranordnungen den Aufschneide- oder Aufteilvorgang vorzunehmen. Sofern man in die Stengelenden ein Energieeintragmittel einbringt, beispielsweise in Form von Aluminium- oder Siliciumdioxyd, besteht darüber hinaus die Möglichkeit die freien Stengelenden aufzusprengen, indem sich das Energieeintragmittel unter Aufbringen von Energie wie Ultraschall, Infrarotwärme oder dergleichen schlagartig ausdehnt und den Aufteilvorgang vornimmt. Auch chemische Aufätzvorgänge können zu der benötigten Vielzahl an Einzelfasern oder Filamenten führen.

Mit dem beschriebenen Haftverschlußteil und dem Verfahren zu seiner Herstellung lassen sich nicht nur in revolutionärer Weise Van-der-Waals-Bindungskräfte einsetzen, um Anhaftvorgänge des Haftverschlußteiles mit beliebigen Oberflächen zu erreichen, sondern es hat sich auch gezeigt, dass ein dahingehender Haftverschluß ähnlich dem Lotus-Effekt selbstabreinigende Wirkung hat, und aufgrund des Mikrocharakters des Verschlußteiles und den eingesetzten Materialien mit niedriger Oberflächenenergie ist gewährleistet, dass Schmutzpartikel insbesondere in flüssiger Form von der künstlichen Haftoberfläche abperlen und diese nicht verschmutzen. Insofern ist mit lang andauernden Einsatzmöglichkeiten für das erfindungsgemäße Haftverschlußteil zu rechnen.

## Patentansprüche

1. Verfahren zum Herstellen eines Haftverschlußteiles, bei dem ein Träger (10) mit einer Vielzahl von mit ihm verbundenen Stengeln (12) erstellt wird, wobei bei dem Haftverschlußteil zumindest ein Teil der freien Enden der Stengel (12) mit einer Vielzahl von Einzelfasern (14) versehen wird, **dadurch gekennzeichnet, dass** der jeweilige Stengel (12) mittels eines Formgebungsverfahrens unter Einsatz einer Siebwalze oder formwerkzeugfrei über ein Tröpfchen-Auftragverfahren erhalten wird, und dass anschließend der jeweilige Stengel (12) an seinem freien Ende in eine Vielzahl von Einzelfasern (14) aufgeteilt wird.

2. Verfahren nach Anspruch 1, **dadurch gekennzeichnet, dass** die Vielzahl von Einzelfasern (15) für jeden Stengel (12) an ihrem freien Ende in eine gemeinsame Ebene münden.

3. Verfahren nach Anspruch 2, **dadurch gekennzeichnet, dass** die Länge der Einzelfasern (14) weniger als die Hälfte, vorzugsweise 10 bis 30 % der gesamten Stengellänge beträgt.

4. Verfahren nach einem der Ansprüche 1 bis 3, **dadurch gekennzeichnet, dass** ungefähr 1000 bis 10.000 Stengel (12), insbesondere 3000 bis 6000, vorzugsweise etwa 4000 Stengel pro Quadratzentimeter Trägerfläche vorgesehen werden, und dass pro Stengel, insbesondere 50 bis 5000, vorzugsweise etwa 500 Einzelfasern (14) vorgesehen werden.

5. Verfahren nach einem der Ansprüche 1 bis 4, **dadurch gekennzeichnet, dass** zum Aufteilen der Einzelfasern (14) aus dem freien Ende der Stiele (12) diese chemisch, mechanisch oder elektrisch (Wärme-) behandelt werden.

6. Verfahren nach einem der Ansprüche 1 bis 5, **dadurch gekennzeichnet, dass** der Träger (10), die Stengel (12) und die Einzelfasern (14) aus einem Kunststoffmaterial bestehen, insbesondere ausgewählt aus der Gruppe Acrylate wie Polymethacrylate, Polyethylen, Polypropylene, Polyoxymethylene, Polyvinyliden-fluorid, Polymethylpenten, Polyethylen-chlorotrifluoroethylen, Polyvinylfluorid, Polyethylenoxid, Polyethylenterephthalate, Polybutylenterephthalate, Nylon 6, Nylon 66 und Polybuten.

7. Verfahren nach einem der Ansprüche 1 bis 6, **dadurch gekennzeichnet, dass** die Einstellparameter für die einzelnen Vorgänge zum Erhalt der Stengel (12) derart eingestellt werden, dass im wesentlichen zylindrische Stengel (12) mit zunächst planen Kopfenden an ihrer Stirnseite erhalten werden.

## Claims

1. A method for producing an adhesive fastener part wherein a support (10) is produced having a plurality of stalks (12) connected to it, with the adhesive fastener part at least some of the free ends of the stalks (12) being provided with a plurality of individual fibres (14), **characterised in that** the respective stalk (12) is obtained by means of a moulding method using a perforated roller or without any moulding tools by means of a droplet application method, and that the respective stalk (12) is then divided on its free end into a plurality of individual fibres (14).

2. The method according to Claim 1, **characterised in that** the plurality of individual fibres (15) lead to a common plane for each stalk (12) on its free end.

3. The method according to Claim 2, **characterised in that** the length of the individual fibres (4) is less than a half, preferably 10 to 30 %, of the total stalk length.

4. The method according to any of Claims 1 to 3, **characterised in that** approximately 1,000 to 10,000 stalks (12), in particular 3,000 to 6,000, preferably approximately 4,000 stalks are provided per square centimetre of support surface, and that in particular 50 to 5,000, preferably approximately 500 individual fibres (14), are provided per stalk.

5. The method according to any of Claims 1 to 4, **characterised in that** for the purpose of division of the individual fibres (14) from the free end of the stalks (12) the latter are subjected to chemical mechanical or electric (heat) treatment.

6. The method according to any of Claims 1 to 5, **characterised in that** the support (10), the stalks (12) and the individual fibres (14) are made of a plastic material, in particular chosen from the group of acrylates such as polymethacrylates, polyethylene, polypropylenes, polyoxymethylenes, polyvinylidene fluoride, polymethylpentene, polyethylene chlorotrifluoroethylene, polyvinyl fluoride, polyethylene oxide, polyethylene terephthalates, polybutylene terephthalates, nylon 6, nylon 66 and polybutene.

7. The method according to any of Claims 1 to 6, **characterised in that** the adjustment parameters for the individual processes for obtaining the stalks (12) are set such that substantially cylindrical stalks (12) with initially planar head ends on their front side are obtained.

## Revendications

1. Procédé de fabrication d'une partie de fermeture autoagrippante, dans lequel on se procure un support (10) ayant une pluralité de tiges (12) qui y sont reliées, dans lequel, dans la partie de la fermeture, au moins une partie des extrémités libres des tiges (12) est munie une pluralité de fibres (14) individuelles, **caractérisé en ce que** l'on obtient la tige (12) respective au moyen d'un procédé de façonnage en utilisant un rouleau formant tamis ou sans outil de moulage par un procédé de dépôt de gouttelettes et **en ce qu'**ensuite on subdivise la tige 12 respective à son extrémité libre en une pluralité de fibres (14) individuelles.

2. Procédé suivant la revendication 1, **caractérisé en ce que** la pluralité de fibres (15) individuelles débouche pour chaque tige (12) à son extrémité libre dans un plan commun.

3. Procédé suivant la revendication 2, **caractérisé en ce que** la longueur des fibres (14) individuelles représente moins de la moitié, de préférence de 10 à 30 %, de la longueur totale de la tige.

4. Procédé suivant l'une des revendications 1 à 3, **caractérisé en ce que** l'on prévoit environ de 1000 à 10 000 tiges (12), notamment de 3000 à 6000, de préférence environ 4000 tiges par centimètre carré de surface du support et **en ce que** l'on prévoit par tige notamment de 50 à 5000, de préférence environ 500 fibres (14) individuelles.

5. Procédé suivant l'une des revendications 1 à 4, **caractérisé en ce que**, pour la subdivision des fibres (14) individuelles de l'extrémité libre des tiges (12), on les traite chimiquement, mécaniquement ou électriquement (à la chaleur).

6. Procédé suivant l'une des revendications 1 à 5, **caractérisé en ce que** le support (10), les tiges (12) et les fibres (14) individuelles sont en une matière plastique, choisie notamment dans le groupe des acrylates, comme le polyméthacrylate, le polyéthylène, le polypropylène, le polyoxyméthylène, le poly(fluorure de vinylidène), le polyméthylpentène, le polyéthylène-chlorotrifluoréthylène, le poly(fluorure de vinyle), le poly(oxyde d'éthylène), le poly(téréphtalate d'éthylène), le poly(téréphtalate de butylène), le nylon 6, le nylon 66 et le polybutène.

7. Procédé suivant l'une des revendications 1 à 6, **caractérisé en ce que** l'on règle les paramètres de réglage des divers opérations d'obtention des tiges (12) de manière à obtenir des tiges (12) sensiblement cylindriques ayant sur leur côté frontale des extrémités de tête d'abord plane.
